# EUROPEAN PATENT APPLICATION

(11) **EP 2 487 185 A1**
(43) Date of publication of application: **15.08.2012**
(21) Application number: 11154195.9
(22) Date of filing: 11.02.2011
(51) Int. Cl.: C07K 14/685, A61K 38/34, A61P 17/16, A61P 35/00

(54) **Hexapeptide with improved activity in the repair of cellular DNA of dermal cells**

(71) Applicant: Clinuvel Pharmaceuticals Limited, Melbourne, VIC 3000 (AU)
(72) Inventor: Wolgen, Philippe, Melbourne Victoria 3000 (AU)
(74) Representative: Farago, Peter Andreas

(57) **Abstract**

The present invention relates to a compound having a structure selected from: Ac-Nle-Glu-His-D-Phe-Arg-Trp-NH₂ (SEQ ID NO: 1); orAc-Nle-Gln-His-D-Phe-Arg-Trp-NH2 (SEQ ID NO: 2) or a pharmaceutically acceptable salt thereof. The compound of the salt thereof are particularly useful for the repair or prevention of damage of cellular DNA of dermal (skin) cells following UV irradiation in a mammal.

## Description

The present invention relates to a hexapeptide having an improved activity in the repair and/or prevention of damage of cellular DNA of dermal (skin) cells following UV irradiation, the improved activity in repair and/or prevention of damage being superior to the physiological molecule alpha-MSH. According to the present invention the hexapeptide having an improved activity is CUV 9900 which will be defined hereinafter.

### Introduction/Background of the Invention

UV radiation, as used for instance in phototherapy, is known to cause damage to dermis and epidermis, whereby the superficial cells acting as first line of defence are keratinocytes and melanocytes. Melanocytes play a critical role in photoprotection of the skin by synthesizing melanin and transferring it to surrounding keratinocytes. Melanin shields epidermal cells from UV rays that damage nuclear DNA and also scavenges reactive oxygen radicals that cause oxidative damage to cellular DNA, proteins and lipids. A major regulator of human pigmentation is the melanocortin alpha-MSH ("MSH" stands for "Melanocyte-stimulating hormone") which binds to the "Melanocortin-1 Receptor" (MCRI) on human melanocytes and thereby stimulates the synthesis of melanin. The activation of MC1R by alpha-MSH also enhances the repair of UV-induced DNA-photoproducts.

UV damage is expressed as DNA damage following the formation of photoproducts such as, for example, 8-oxo-2'-deoxyguanosine (8-oxo-dG) and cyclobutane pyrimidine dimers (CPD). 8-Oxo-dG is an oxidized derivative of deoxoguanosine and is one of the major products of DNA oxidation. Concentrations of 8-oxo-dG within a cell are a measurement of oxidative stress. Pyrimidine dimers are molecular lesions formed from thymine or cytosine bases in DNA via photochemical reactions. These premutagenic lesions alter the structure of DNA and consequently inhibit polymerases and arrest replication. Dimers may be repaired by photoreactivation or nucleotide excision repair, but unrepaired dimers are mutagenic. In humans the mutagenicity may cause to a number of skin cancers.

Accordingly, there is an increasing interest in developing melanocortin analogues that both stimulate the synthesis of melanin (induce sunless tanning providing photoprotection) and enhance the repair of UV-induced DNA-photoproducts.

### Summary of the Invention

In one aspect, the present invention relates to a compound, hereinafter referred to as CUV99000, having a structure selected from:
Ac-Nle-Glu His-D-Phe-Arg-Trp-NH₂ (SEQ ID NO: 1); and
Ac-Nle-Gln His-D-Phe-Arg-Trp-NH₂ (SEQ ID NO: 2)
or a pharmaceutically acceptable salt thereof.

In another aspect, the present invention relates to a pharmaceutical composition comprising a compound of the present invention, or a pharmaceutically acceptable salt thereof, in combination with a pharmaceutically acceptable carrier.

In another aspect, the present invention relates to a rod, microcapsule or implant comprising
a) the compound of the present invention; or
b) the pharmaceutical composition of the present invention;
in combination with a biodegradable polymer, wherein the amount of the compound is from 5% to
60% by weight of the rod, microcapsule or implant.

In another aspect, the present invention relates to a compound of the present invention, or a pharmaceutically acceptable salt thereof, for use in stimulating the synthesis of melanin in a mammal.

In another aspect, the present invention relates to a compound of the present invention, or a pharmaceutically acceptable salt thereof, for use in the repair and/or prevention of damage of cellular DNA of dermal (skin) cells following UV irradiation in a mammal. Such UV radiation can be induced for instance by phototherapy (nUVA and nUVB) in dermatology in which UV is comprised of UVA and UVB with a typical UVB:UVA ratio of 75%:25%.

In another aspect, the present invention relates to a compound of the present invention, or a pharmaceutically acceptable salt thereof, for use in the repair and/or prevention of damage of cellular DNA of dermal (skin) cells without UV irradiation, with the compound being in the form of a night cream.

In another aspect, the present invention relates to a compound of the present invention, or a pharmaceutically acceptable salt thereof, for use in treating cancer, preferably melanoma, in a mammal.

### Brief Description of the Drawings

Fig. 1 shows the steps in the synthesis of CUV9900 in the Ac-Nle-Glu His-D-Phe-Arg-Trp-NH₂ (SEQ ID NO: 1) variation thereof;
Fig. 2 shows a CPD median fluorescence intensity of a 3-5 determinations/group ±S.E for the Ac-Nle-Glu His-D-Phe-Arg-Trp-NH₂ (SEQ ID NO: 1) variation of CUV9900 as compared to alpha-MSH and NDP-MSH;
Fig..3 shows the potency of the Ac-Nle-Glu His-D-Phe-Arg-Trp-NH₂ (SEQ ID NO: 1) variation of CUV9900 as compared to alpha-MSH and NDP-MSH for a melanocytes strain 1513c;
Fig. 4 shows the potency of the Ac-Nle-Glu His-D-Phe-Arg-Trp-NH₂ (SEQ ID NO: 1) variation of CUV9900 as compared to NDP-MSH for a melanocytes strain1505b;
Fig. 5a shows the potency of the Ac-Nle-Glu His-D-Phe-Arg-Trp-NH₂ (SEQ ID NO: 1) variation of CUV9900 as compared to alpha-MSH and NDP-MSH for a melanocytes strain 1505b;
Fig. 5b shows the potency of the Ac-Nle-Glu His-D-Phe-Arg-Trp-NH₂ (SEQ ID NO: 1) variation of CUV9900 as compared to alpha-MSH and NDP-MSH for a melanocytes strain 105c;
Fig. 6 shows the potency of the Ac-Nle-Glu His-D-Phe-Arg-Trp-NH₂ (SEQ ID NO: 1) variation of CUV9900 as compared to alpha-MSH and NDP-MSH for a melanocyte strain 102b;
Fig. 7 shows the stability the Ac-Nle-Glu His-D-Phe-Arg-Trp-NH₂ (SEQ ID NO: 1) variation of CUV9900 versus that of NDP-MSH and alpha-MSH for the melanocyte strain 1513c;
Fig. 8 shows the stability the Ac-Nle-Glu His-D-Phe-Arg-Trp-NH₂ (SEQ ID NO: 1) variation of CUV9900 versus that of NDP-MSH and alpha-MSH for the melanocyte strain 1505b;
Fig. 9 shows the stability the Ac-Nle-Glu His-D-Phe-Arg-Trp-NH₂ (SEQ ID NO: 1) variation of CUV9900 versus that of NDP-MSH and alpha-MSH for the melanocyte strain 1513c, however 6 months later than the experiment of Fig. 7;
Fig. 10 shows hydrogen peroxide generation for the Ac-Nle-Glu His-D-Phe-Arg-Trp-NH₂ (SEQ ID NO: 1) variation of CUV9900 versus that of NDP-MSH and alpha-MSH for the melanocyte strain 1505b;
Fig. 11 shows hydrogen peroxide generation for the Ac-Nle-Glu His-D-Phe-Arg-Trp-NH₂ (SEQ ID NO: 1) variation of CUV9900 versus that of alpha-MSH for the melanocyte strain 103c;
Fig. 12 shows the measurement of UV-induced apoptosis by Annexin Va staining for the Ac-Nle-Glu His-D-Phe-Arg-Trp-NH₂ (SEQ ID NO: 1) variation of CUV9900 versus that of NDP-MSH, alpha-MSH and Forskolin for the melanocyte strain 103c;
Fig. 13 shows the measurement of UV-induced apoptosis by Annexin Va staining for the Ac-Nle-Glu His-D-Phe-Arg-Trp-NH₂ (SEQ ID NO: 1) variation of CUV9900 versus that of NDP-MSH and Forskolin for the melanocyte strain 1513c;
Fig. 14 is a ultrastructural visualization of DOPA-stained HM for the Ac-Nle-Glu His-D-Phe-Arg-Trp-NH₂ (SEQ ID NO: 1) variation of CUV9900 versus that of alpha-MSH for the lightly pigmented strain 1563c; and
Fig. 15 is a ultrastructural visualization of DOPA-stained HM for the Ac-Nle-Glu His-D-Phe-Arg-Trp-NH₂ (SEQ ID NO: 1) variation of CUV9900 versus that of alpha-MSH for the102b strain;

### Detailed Description of the Invention

In the following, the invention will be set out with reference to the detailed description hereinbelow and attached figures described above.

The present invention provides for a hexapeptide, namely CUV 9900, which is an alpha-MSH analogue capable of stimulating the melanocytes and consequently, the synthesis of melanin. Furthermore, it has been shown that CUV9900 is a potent skin protectant and has prolonged activity in the repair of cellular DNA of dermal (skin) cells following UV irradiation. CUV 9900 is superior to the physiological molecule alpha-MSH and can be beneficial in many disorders, for example in disorders where the melanogenic response is deficient in its activity due to insufficient signalling following alpha-MSH release, or in disorders where melanogenesis is deficient due to metabolic, immunogenic, genetic or mechanical factors affecting the biological signalling of the melanocyte.

According to the present invention CUV9900 has the structure:
Ac-Nle-Glu His-D-Phe-Arg-Trp-NH₂ (SEQ ID NO: 1) or
Ac-Nle-Gln His-D-Phe-Arg-Trp-NH₂ (SEQ ID NO: 2).

Preferably, CUV9900 is fully water soluble as acetate salt and lyophilized, and has a molecular weight of 927 gram/mol.

The alpha-MSH which is used in the comparative examples of the present invention has the structure: Ac-Ser-Tyr-Ser-Met-Glu-His-Phe-Arg-Trp-Gly-Lys-Pro-Val-NH₂ (SEQ ID NO: 3). Whenever the terms MSH or α-MSH are used in the description or figures it is intended to designate the above defined alpha-MSH.

NDP-MSH is a synthetic analog of alpha-MSH which is also used in the comparative examples of the present invention and has the structure: Ac-Ser-Tyr-Ser-Nle-Glu-His-D-Phe-Arg-Trp-Gly-Lys-Pro-Val-NH₂ (SEQ ID NO: 4). Whenever the terms NDPαMSH or afamelanotide are used in the description or figures it is intended to designate the above defined NDP-MSH.

All peptide sequences are written according to the generally accepted convention whereby the alpha-N-terminal amino acid residue is on the left and the alpha-C-terminal is on the right.

| **Abbreviation** | **Definition** |
|---|---|
| Ac | Acetyl |
| Ser | Seryl |
| Tyr | Tyrosyl |
| Met | Methionyl |
| Nle | Norleucyl |
| Glu | Glutamyl |
| His | Histidyl |
| Phe | Phenylalanyl |
| Trp | Tryptyl |
| Gly | Glycyl |
| Lys | Lysyl |
| Pro | Prolyl |
| Val | Valyl |

### Synthesis of the peptide

One currently preferred method for manufacturing the Ac-Nle-Glu-His-D-Phe-Arg-Trp-NH₂ (SEQ ID NO: 1) variation of CUV9900 is described hereinbelow. However the person skilled in the art will appreciate that alternatives methods for the manufacturing of said variation of CUV900 can be implemented. The manufacturing of the Ac-Nle-Gln-His-D-Phe-Arg-Trp-NH₂ (SEQ ID NO: 2) variation of CUV9900 can be implemented in a similar manner as will be appreciated by the person skilled in the art.

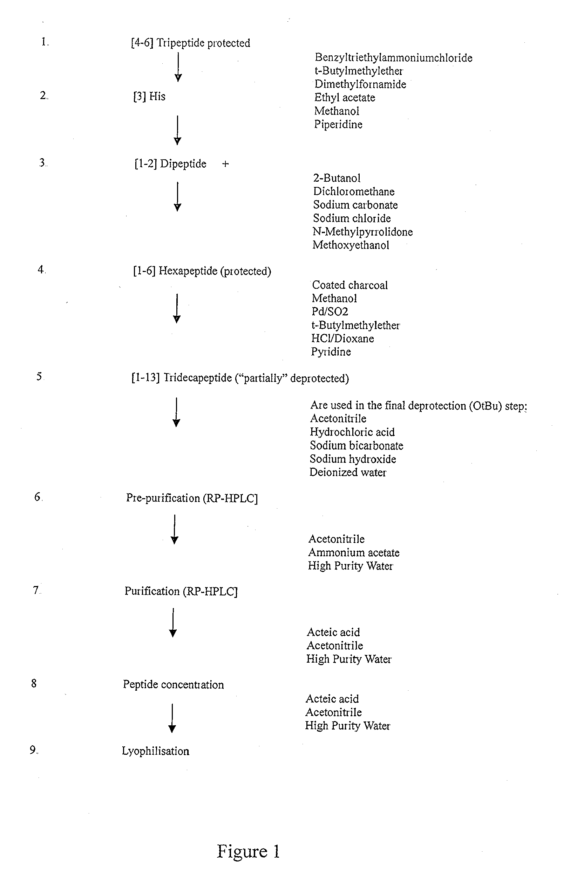

According to a currently preferred method, CUV9900 in the variation Ac-Nle-Glu-His-D-Phe-Arg-Trp-NH₂ (SEQ ID NO: 1) can be manufactured by solution-phase peptide synthesis, followed by chromatographic purification and lyophilisation. With reference to **Fig. 1** the steps in the peptide synthesis are:
- The protection of tripeptide [4-6] is the initial step of synthesis
- The protected tripeptide [4-6] and fluorenylmethoxycarbonyl- (Fmoc) and trityl- (Trt) protected [3] histidine (His) is added to form the tetra peptide [6-9]
- The dipeptide Nle-Glu and tetrapeptide [6-9] are coupled to produce the protected hexapeptide CUV9900[1-6].

The final steps in the peptide synthesis are summarised below and illustrated in **Fig. 1****.** The key intermediates in the synthesis of CUV9900 are the [1-2] and the [4-6] peptide fragments while the final intermediate is the protected [1-6] hexapeptide with the final amino acid (aa) sequence. The final coupling of the aforementioned peptide fragments and subsequent de-protection steps are summarized below and are also shown in **Fig. 1****.**

### Final Coupling

The final coupling consists of the condensation of the [1-2] dipeptide with the [3-6] tetrapeptide to form protected CUV9900.

### De-protection

The two protecting groups trityl (Trt) on the His residue at position 3 and O-tert-butyl (OtBu) on the Glu residue at position 2 are removed from the protected CUV9900.

### Purification

The resulting peptide solution then undergoes the following multi-step purification process:
- **Pre-purification.** This step uses a preparative C18 reverse phase HPLC (RP-HPLC) column and an acetonitrile/ammonium acetate buffered aqueous mobile phase. Mostly salt and some synthetic impurities are removed by this step.
- **Purification**. The pooled pre-purification fractions are diluted with a mixture of high purity water and acetic acid and purified by preparative RP-HPLC using a C 18 column and an acetic acid/acetonitrile/water mixture gradient mobile phase using increasing concentrations of acetonitrile.
- **Concentration**. The same chromatographic column as used during purification is then used to concentrate the peptide solution with an acetic acid/acetonitrile/water mobile phase.

Organic solvents are removed by evaporation before the concentrated aqueous peptide solution is freeze dried.

### Biological Activity of CUV9900 / Examples

In all following examples the Ac-Nle-Glu His-D-Phe-Arg-Trp-NH₂ (SEQ ID NO: 1) variation of CUV9900 was used. However, a person skilled in the art will appreciate that the Ac-Nle-Gln His-D-Phe-Arg-Trp-NH₂ (SEQ ID NO: 2) variation of CUV9900 will induce substantially the same biological activity.

For all experiments, melanocytes were deprived of the bovine pituitary extract, a supplement of the melanocyte growth medium which contains micro- and millimolar concentrations of melanocortins, in order to allow for cellular responsiveness to agonistic melanocortins.

CUV 9900 in the variation explained hereinabove, namely Ac-Nle-Glu His-D-Phe-Arg-Trp-NH₂ of SEQ ID NO: 1, was tested versus alpha-MSH (the physiological MSH) of SEQ ID NO: 3 and NDP-MSH (afamelanotide) of SEQ ID NO: 4, wherein the respective structures of alpha-MSH and NDP-MSH were as indicated above.

In all examples the UV emitted and used to irradiate cell cultures were represented by the ratio UVB:UVA ratio 75%:25%.

### Example 1

### DNA damage as measured by 8-oxo-2'-deoxyguanosine (8-oxo-dG)

DNA damage of cultured melanocytes following UV irradiation leads to consistent levels of photoproducts such as 8-oxo-dG. It is well established in the art (dermal carcinogenesis) that 8-oxodG is a major form of UV induced oxidative DNA damage, which results from the generation of reactive oxygen species (ROS). The induction of 8-oxodG by 8-oxo-dGTP can be inhibited by pyrophosphohydrolases (8-oxo-dGTPases) that convert it to 8-oxo-dGMP. To elucidate the involvement of 8-oxo-dGTPases in carcinogenesis, an assay of the 8-oxo-dGTPase activity was used (Sigma Chemical Co. (USA) as well as a conjugated 8-oxo-dG antibodies (OxoDNA Assay Kit; Calbiochem/EMD Biosciences).

Melanocytes were plated onto coverslips, and treated 48 h thereafter for 4 days prior to, and immediately after irradiation with 0 or 105 mJ/cm² UV, 0.1-10.0 nM α-MSH, with 0 (control) or 0.1-10.0 nM α-MSH, or 0.001-10 nM NDP-MSH or 0.001-10.0 nM CUV 9900. Two hours after UV exposure, cells were fixated with 4% paraformaldehyde in PBS, hydrated in increasing concentrations of cold methanol, and permeabilized in 99% methanol for 30 min at 4 °C. Melanocytes were incubated in blocking solution at 37 °C for 1 h and then with FITC-conjugated 8-oxo-dG antibody, or mouse IgG as a negative control, at 37 °C for 1 h. The IgG controls were then incubated with FITC-conjugated anti-mouse antibody. Immunofluorescence was detected using a microscope with FITC filters. Data were expressed as the mean fluorescence intensity ±S.E., determined using the densitometry values from 6-8 randomly obtained images, using the computer assisted program Alpha Inotech Imaging System and the AlphaEase FC StandAlone Software.

As an expected benchmark, UV irradiation alone gives rise to values of up to 80 "arbitrary units" (a.u.) and a standard deviation of approximately 20. The experiments show that physiological alpha-MSH following UV irradiation gives rise to values of 40 to 45 a.u. and a standard deviation of approximately 10. In comparison, the potency of CUV9900 alone in the experiments conducted gave rise to 30 a.u. of 8-oxo-dG and a standard deviation of 10, while UV in combination with CUV9900 resulted in values of 38 and a standard deviation of 10. In comparison, the potency of NDP-MSH is somewhat higher following UV irradiation whereby values of 39-40 a.u. were found and a standard deviation of approximately 10.

Due to the longer half-life of CUV9900 effectuated by the change of aminocids at position 1 and 4, it is well established in the art that -alike NDP-MSH, where at aminoacids were changed at position 4 and 7 - melanocortin-1 receptor binding and dissociation is stronger than seen with alpha-MSH. Due to the increased potency of CUV9900 and expected clinical effect these results confirm the ability of CUV9900 to reduce, mitigate and abrogate photodamage.

In conclusion, the potency of CUV9900 gave 33% less 8-oxo-dG cellular DNA damage in comparison with alpha-MSH and 120% less damage than UV alone.

| Experimental Agent | Source | Dose | Incubation Temp. (Celsius) | Substrate | Arbitrary units | Standard Deviation |
|---|---|---|---|---|---|---|
| None | 75%UVB-25% UVA | 10.5mJ/cm² | 37 | human melanocytes | 80 | 20 |
| Alpha-MSH | None | - | 37 | human melanocytes | 40-45 | 10 |
| CUV9900 | None | - | 37 | human melanocytes | 30 | 10 |
| CUV9900 | 75%UVB 25% UVA | 105 mJ/cm² | 37 | human melanocytes | 38 | 10 |
| NDP | 75%UVB 25% UVA | 105 mJ/cm² | 37 | human melanocytes | 39-40 | 10 |

### Example 2

### DNA damage as measured by cyclobutane pyrimidine dimers (CPD)

Cyclobutane pyrimidine dimers (CPD), the major determinant of UV induced DNA photoproducts, were detected using flow cytometry analysis.

Melanocytes were pretreated with 0 (control), 10 nM α-MSH or 1 nM NDP-MSH or 1 nM CUV9900 for 4 days prior to, and 2 days after exposure to 105 mJ/cm² UV, harvested 48 hours post irradiation, fixated with 70% ethanol, the DNA denatured with 1N HCl/0.2 mg/ml pepsin, then renaturated with sodium tetraborate. Melanocytes were blocked and incubated overnight at 4°C with the anti-CPD antibody (diluted 1:500; TDM-2 clone), then with goat anti-mouse IgG Alexa Fluor 488 (Invitrogen, Carlsbad CA) for 1 hour, and finally resuspended in PBS containing RNase A and propidium iodide. Melanocytes were analyzed on a Coulter EPICS XL flow cytometer (Beckman Coulter) using a 488nm argon ion laser A 525 band pass filter was used for the Alexa fluor 488 and a 620 band pass filter for the propidium iodide.

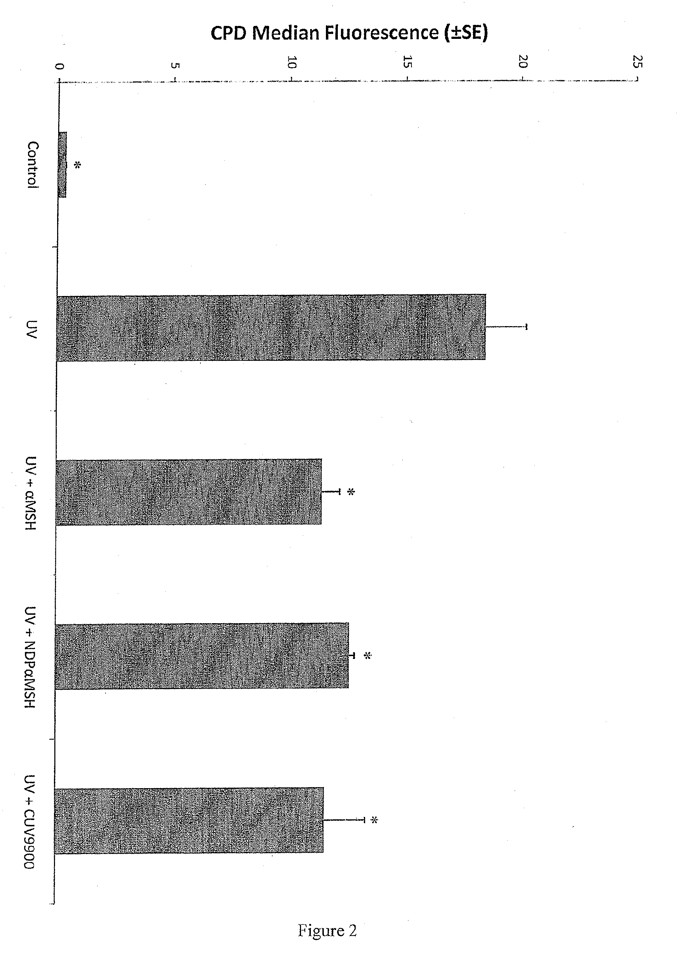

Data are expressed as CPD median fluorescence intensity of 3-5 determinations/group ±S.E and shown in **Fig. 2**.

In the current experiments, CPD levels were measured 48 h after UV exposure to determine the effects of α-MSH and the analog on CPD repair. Absolute values of CPD products following UV irradiation were approximately 18 following UV irradiation alone, 11.5 for UV and alpha-MSH, 12.5 for UV and NDP-MSH, and 11.55 for UV and CUV9900.

Overall, treatment with CUV 9900 resulted in less CPD cellular DNA damage in comparison with and NDP-MSH and slightly higher values than alpha-MSH. However, it is established that physiological alpha-MSH harbours a short half-life clinically, rendering the molecule in humans highly ineffective. In these results, it is seen that due to the stronger binding affinity and longer half-life in comparison with alpha-MSH, CUV9900 represents a more effective and potent molecule in assessing specific cellular repair on the basis of equimolar concentrations.

### Example 3

### Dose dependent effects of CUV9900 compared to alpha-MSH and NDP-MSH, on cyclic adenosine 3',5'-cyclic monophosphate (cAMP) and tyrosinase activity of cultured human melanocytes

Melanocytes respond to melanocyte-stimulating hormone (MSH), acting through cAMP, by demonstrating increased activity of tyrosinase, the rate-limiting enzyme for melanin synthesis. Accordingly, tyrosinase activity is indicative for the effectivity and functionality of the melanocyte.

### Determination of cAMP levels:

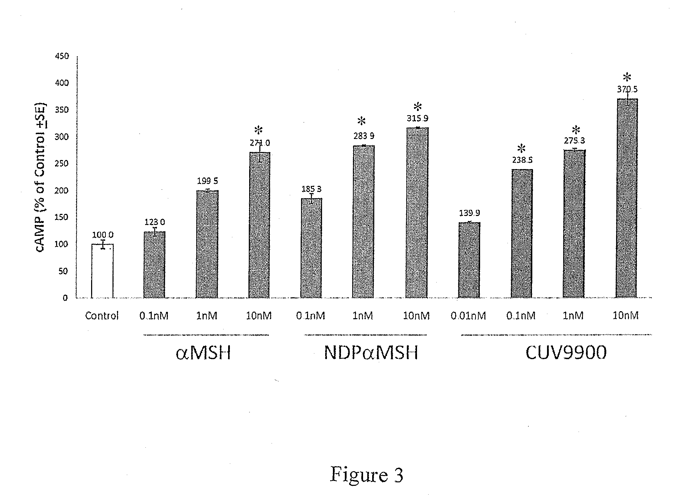

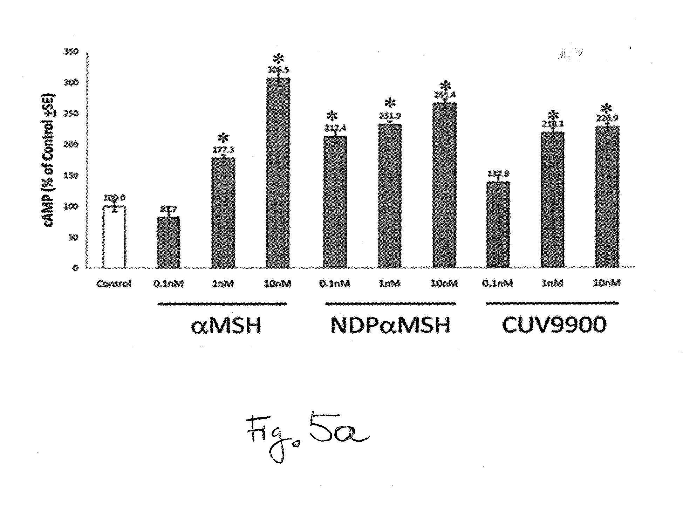

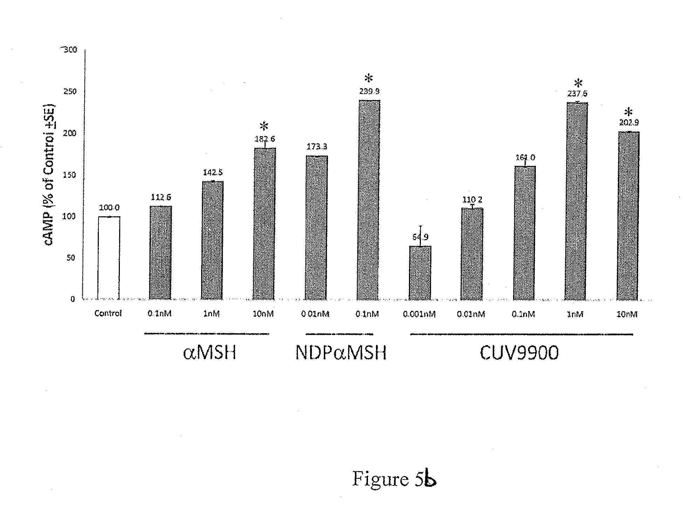

Dose-response experiments were carried out on three melanocytic strains, namely strains 1513c, 1505b and 105c melanocytes were treated with 1 pM-10 nM CUV9900 or NDP-MSH, or with 0.1-10 nM MSH for 45 minutes in the presence of 0.1 mM isobutyl methylxanthine (IBMX). The results obtained from strain 1513c are shown in Fig. 3, the results obtained from strain 1505b are shown in Fig. 5a and the results obtained from strain 105c are shown in Fig. 5b. The strains 1505b and 1513c were obtained from the same human adult while the strain 105c was obtained from another human adult.

Cyclic AMP is the second messenger for the melanocortin-1 receptor, and assessment is usually performed indicating cellular activity reflective of melanogenesis. Cyclic AMP levels were determined using ¹²⁵I-labeled radioimmunoassay kit (Perkin Elmer, Waltham, MA), in human melanocytes that were treated with 0 (control), 0.1-10.0 nM α-MSH, or 0.001-10 nM NDP-MSH or 0.001-10.0 nM CUV 9900 for 45 min. in the presence of 0.1 mM IBMX, a phosphodiesterase inhibitor to prevent cAMP degradation. Triplicate wells were included in each group, and duplicate samples from each well were assayed for cAMP levels. Each data point is the mean percent of control of 6 determinations/group ±S.E.

It was found, as apparent from Fig. 3, that in the 1513c strain, at 0.1 nM concentrations, CUV9900 was more potent than NDP-MSH, and at 10 nM both achieved a higher maximal level of cAMP than MSH. This was statistically different from control in a two-tail test, as determined by ANOVA (p<0.05).

It was found, as apparent from Fig5a, that in 1505b strain, at 1 nM concentrations, CUV9900 was more potent than MSH.

In the adult 105c strain, as shown in Fig. 5b, the result of the test with concentrations of 0.1 nM, 1 nM and 10 nM CUV9900 were significantly higher than 0.1 nM, 1 nM and 10 nM MSH. This was statistically different from control in a two-tail test, as determined by ANOVA (p<0.05).

In this particular strain, at 0.01 nM and 0.1 nM concentrations CUV9900 caused less elevation of cAMP in comparison to 0.01 nM and 0.1 nM NDP.

### Tyrosinase activity:

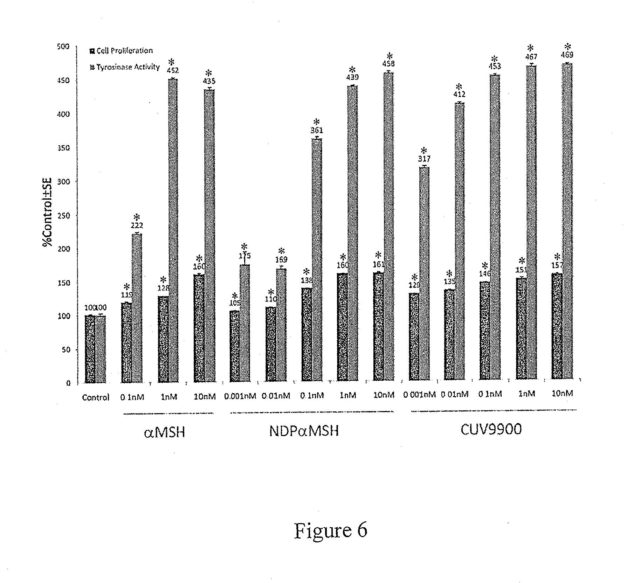

Dose-response experiments were carried out on another adult strain 102b which is shown in Fig. 6 and on the strain 1505b which is a melanocyte strain: melanocytes were treated with 0.001-10 nM CUV9990 or NDP-MSH, or 0.1-10 nM MSH for a total of 6 days, with the culture medium changed and fresh peptide added every other day. Cell numbers as well as tyrosinase activity were then determined. Tyrosinase activity was calculated first at activity/10⁶ cells, and then as % of control. Twenty-four hours prior the end of the experiment, melanocytes received ³H-tyrosinase (0.7 µCi/ml medium). The conditioned medium from each dish was collected to be assayed for tyrosinase activity, and the cell numbers were determined using a Coulter Counter. Triplicate dishes were included in each group, and duplicate samples from each dish were assayed for tyrosinase activity. Each data point is the mean percent of control of 6 determinations/group ±S.E.

To determine the residual effect of CUV9900, as compared to NDP-MSH and α-MSH, on tyrosinase activity, melanocytes were treated with either CUV9900 or NDP-MSH at the concentrations previously described for a total of 3 days. Tyrosinase activity was measured at the end of the 3 days of treatment, and 2 and 4 days after maintenance of the melanocytes in the complete absence of any treatment.

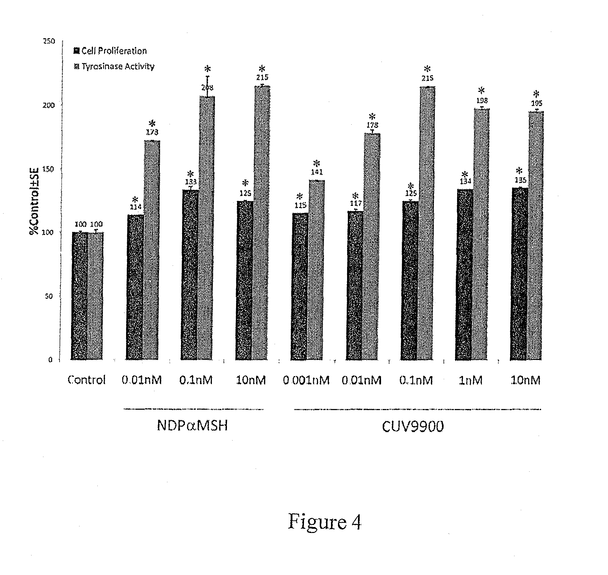

Thus, Figs. 3 to 6 demonstrate the potency of CUV9900 in comparison to NDP-MSH and alpha-MSH as determined in all the samples analyzed.

In the 102b strain, the minimal effective dose of CUV9900 and NDP-MSH was 0.001 nM. CUV9900 resulted in greater stimulation of tyrosinase activity than NDP-MSH at subnanomolar concentrations. Equal maximal stimulation of tyrosinase was achieved with 0.1 nM CUV9900, and 1 nM NDP-MSH and alpha-MSH. This was statistically different from the control values observed, as determined by ANOVA (p<0.05).

In the 1505b strain, the minimal effective dose of CUV9900 was 0.001 nM, and its effect was equivalent to that of NDP-MSH. This was statistically different from control, as determined by ANOVA (p<0.05).

These results demonstrate that at lower concentrations of CUV9900 led to a more significant effect on cellular (melanocyte) activity, as reflected in key enzymatic activity and resulting in more effective cellular repair and melanogenic machinery in comparison to the physiological MC1R agonist MSH.

### Example 4

### Stability of CUV9900 versus that of NDP-MSH and α-MSH

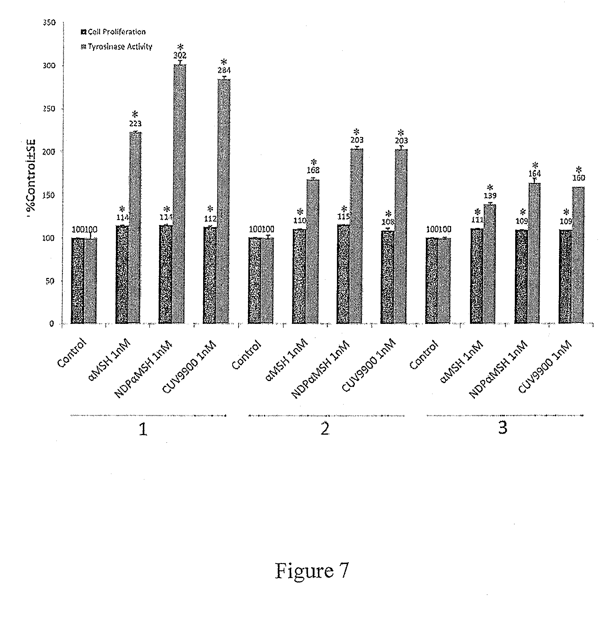

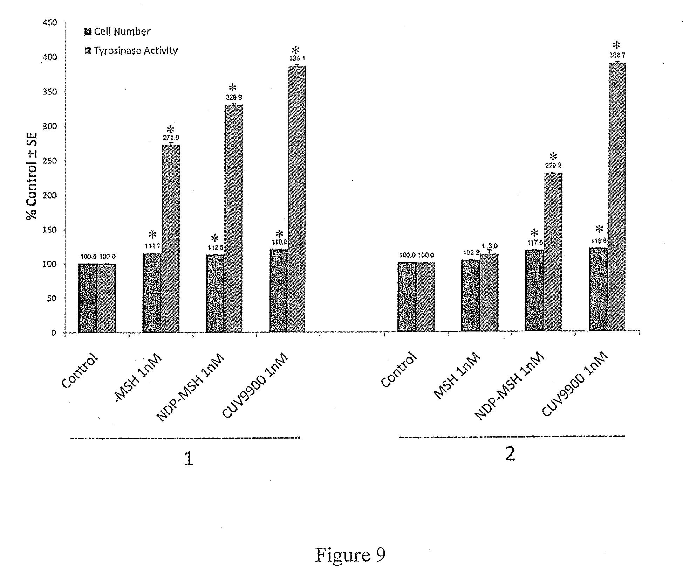

With continued reference to Figs. 7, 8 and 9, to determine the stability of CUV9900 versus that of NDP-MSH and α-MSH, melanocytes were treated with 1 nM of each peptide, namely with CUV9900, NDP-MSH and α-MSH, for a total of 6 days, or with 1 nM peptides that were incubated for 5 hours at 37 °C prior to addition to the cultures, every other day, for a total of 6 days. Tyrosinase activity was measured at the end of treatment. In the experiments, cell number represents the mean percent of control of 3 determinations/ group ±SE, and tyrosinase activity data represent the mean percent of control of 6 determinations ±S.E. It should be noted that for the sake of reproducibility the experiment of strain 1513c of Fig. 9 was performed once more 6 months later than the experiment of Fig. 7.

The analysis of the residual effects of CUV9900 compared to those of NDP-MSH and MSH were carried out in two experiments, namely on strains 1513c and 1505b, as shown in Fig. 7, 8 and 9, respectively. Melanocytes were treated for 3 days with the designated concentrations of CUV9900, NDP-MSH, or MSH, then maintained in absence of peptides for 4 days. Tyrosinase activity was determined 3 days after treatment.

In strain 1513c, the residual effects of CUV9900 were compared 2 and 4 days after removal of peptides. After 3 days of treatment, the effects of CUV9900 and NDP-MSH (284% and 302% of control, respectively) were markedly greater than those of MSH (223% of control). These effects decreased gradually, and 4 days after peptide removal, the residual effect of MSH was 139% of control, and the effects of CUV9900 and NDP-MSH were 160 and 164% of control respectively. This was statistically different from control, as determined by ANOVA (p=<0.05).

Comparison of stability of CUV9900, NDP-MSH and MSH was compared in 1513c, as shown in Fig. 7; the strain was treated for 6 days with 1 nM CUV9900, NDP-MSH or MSH with fresh medium and peptide replenished every 2 days. Melanocytes were treated according to the same experiment protocol but each peptide was incubated for 5-7 hours at 37 degrees Celsius. Incubation of 1 nM MSH totally abolished its effect on tyrosinase activity (330% of control with fresh peptide vs. 229% of control with incubated peptide). Incubation of CUV9900 had no effect on its ability to stimulate tyrosinase activity, suggesting its maintained stability. The assessment of tyrosinase was statistically different from control, as determined by ANOVA (p=<0.05).

Peptides were incubated at 37 degrees Celcius for 5 hours and then split into groups. Cell proliferation was a measure of stability, whereby the experiments on strain 1513c were divided in period 1 and 2 under standard conditions.

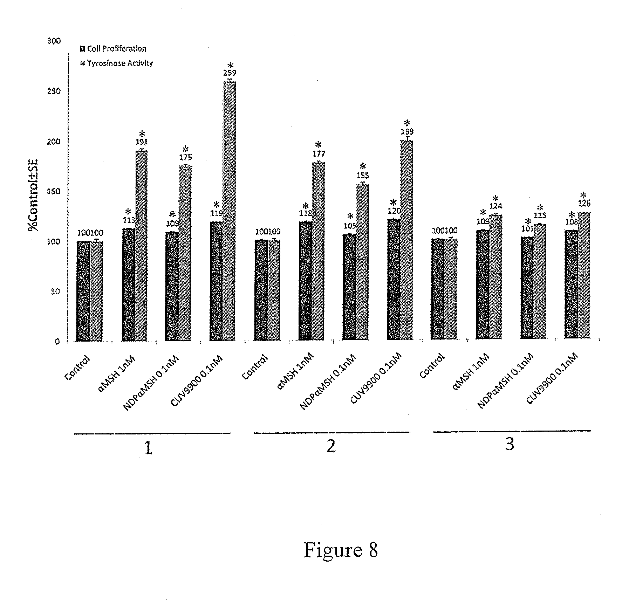

In strain 1505b, as shown in Fig. 8, the residual effects of 0.1 nM CUV9900 and NDP-MSH were compared to that of 1 nM MSH. Although the effect of CUV9900 was markedly greater than that of MSH after 3 days of treatment (259% vs. 191% of control), the residual effects 4 days after peptide removal were equal.

### CELL PROLIFERATION AS A MEASURE OF STABILITY

Cell proliferation as a measure of stability is shown in Tables 1 to 3 below, wherein Table 1 is based on data derived from Fig. 7, Table 2 is based on data derived from Fig. 9, and Table 3 is based on data derived from Fig. 8:

**Table 1**

| STRAIN 1513c [1] | | | | |
|---|---|---|---|---|
| | 1 | 2 | 3 | Mean |
| Control | 100 | 100 | 100 | 100 |
| α-MSH [1nM] | 114 | 110 | 111 | 111.7 |
| NDP-MSH [1nM] | 114 | 115 | 109 | 112.7 |
| CUV9900 [1nM] | 115 | 108 | 109 | 110.7 |

**Table 2**

| STRAIN 1513c [2] | | | |
|---|---|---|---|
| | | | Mean |
| 1 | 2 | Mean | [1+2] |
| 100 | 100 | 100 | 100 |
| 114.7 | 103.2 | 109.0 | 110.6 |
| 112.5 | 117.5 | 115.0 | 113.6 |
| 119.9 | 119.8 | 119.9 | 114.3 |

**Table 3**

| STRAIN 1505b | | | | |
|---|---|---|---|---|
| | 1 | 2 | 3 | Mean |
| Control | 100 | 100 | 100 | 100 |
| α-MSH [1nM] | 113 | 118 | 109 | 113.3 |
| NDP-MSH [1nM] | 109 | 105 | 101 | 105.0 |
| CUV9900 [1nM] | 119 | 120 | 108 | 115.7 |

It is shown from these experiments that CUV9900 demonstrates a preferred stability in comparison to NDP and MSH at equimolar concentrations. In both melanocyte strains CUV9900 achieved optimum stability. In strain 1513c, CUV9900 was 0.65% more stable than the potent agonist NDP-MSH and 3.4% more than MSH. Significantly in 1505b, CUV9900 was 10.16% more stable than NDP-MSH and 2.6% in comparison to MSH. Overall as assessed over all experiments on both strains 1505b and 1513c, CUV9900 showed more cell proliferation and stability than the 2 other compounds NDP-MSH and MSH, respectively 4.0% and 2.9%. The assessment of cell proliferation and inferred stability by CUV9900 was statistically different from control, as determined by ANOVA (p=<0.05).

### TYROSINASE ACTIVITY AS A MEASURE OF STABILITY

Tyrosinase activity as a measure of stability is shown in Tables 4 to 6 below, wherein Table 4 is based on data derived from Fig. 7, Table 5 is based on data derived from Fig. 9, and Table 6 is based on data derived from Fig. 8:

**Table 4**

| STRAIN 1513c [1] | | | | |
|---|---|---|---|---|
| | 1 | 2 | 3 | Mean |
| Control | 100 | 100 | 100 | 100 |
| α-MSH [1nM] | 223 | 168 | 139 | 176.7 |
| NDP-MSH [1nM1 | 302 | 203 | 164 | 223.0 |
| CUV9900 [1nM] | 284 | 203 | 160 | 215.7 |

**Table 5**

| STRAIN 1513c [2] | | | |
|---|---|---|---|
| | | | Mean |
| 1 | 2 | Mean | [1+2] |
| 100 | 100 | 100 | 100 |
| 271.9 | 113 | 192.5 | 183.0 |
| 329.9 | 229.2 | 279.6 | 245.6 |
| 385.1 | 388.7 | 386.9 | 284.2 |

**Table 6**

| STRAIN 1505b | | | | |
|---|---|---|---|---|
| | 1 | 2 | 3 | Mean |
| Control | 100 | 100 | 100 | 100 |
| α-MSH [1nM] | 191 | 177 | 124 | 164.0 |
| NDP-MSH [1nM] | 175 | 155 | 115 | 148.3 |
| CUV9900 [1nM] | 259 | 199 | 126 | 194.7 |

It is seen in the above Table 4 that CUV9900 shows increased tyrosinase activity in comparison to Control and MSH, but marginally less than NDP-MSH in the first experiment in strain 1513c. Over the 2 experiments in strain 1513c reported in the above Table 5, CUV9900 showed to have superior stability over MSH and NDP, respectively 55.3% and 15.7%. The assessment of tyrosinase activity and inferred stability by CUV9900 was statistically different from MSH and NDP, as determined by ANOVA (p=<0.05).

Using strain 1505b, CUV9900 demonstrated superior tyrosinase activity in comparison to MSH and NDP-MSH, respectively 18.7% and .32.1%. Overall, CUV9900 induced superior tyrosinase activity in comparison to MSH and NDP-MSH, respectively 42.5% and 19.8%. All results were found to be statistically different from MSH and NDP-MSH, as determined by ANOVA (p=<0.05).

### Example 5

### Measurement of UV-induced generation of hydrogen peroxide

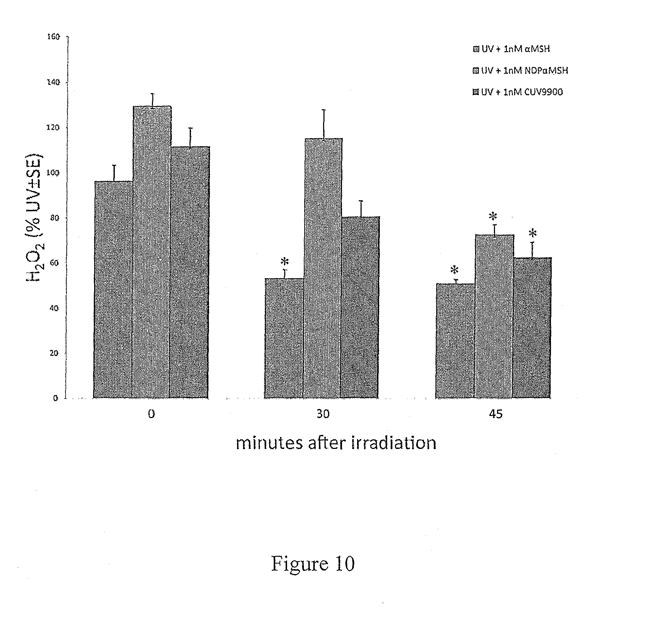

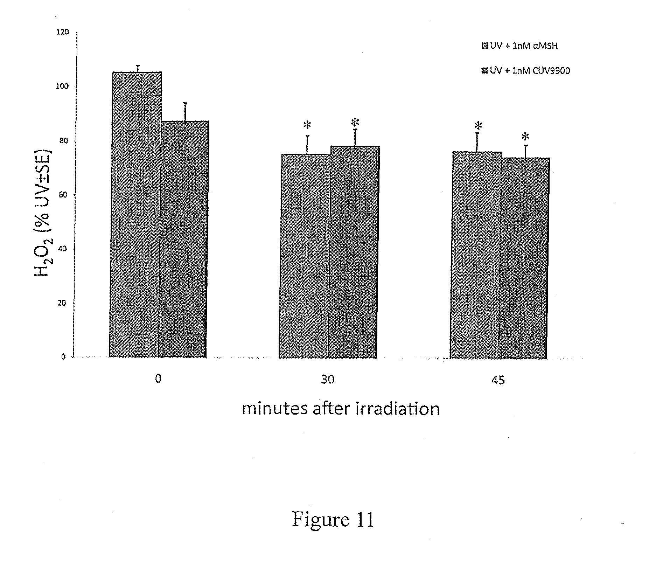

With reference to Figs. 10 and 11, melanocytes were irradiated with a bank of FS 20 lamps, with 75% emission in the UVB, and 25% emission in the UVA spectra, with peak emission at 313 nm wavelength. Any emission of UVC rays from the UV source (FS 20 lamps) was blocked by Kodacel filter. Prior to irradiation, the culture medium was replaced by PBS, which was removed after UV exposure, and fresh medium added.

Melanocytes were irradiated with 105 mJ/cm² UV, and treated immediately while in PBS with 0, 1 nM α-MSH or 1 nM CUV9900. Generation of hydrogen peroxide, representative of reactive oxygen species (ROS), was determined by measuring the luminescence of luminol at 0, 30, and 45 min after UV exposure. Triplicate dishes were included in each group, and duplicate samples from each dish were used for measurement of hydrogen peroxide at each time point. Each data point represents the mean of 6 determinations/group ±S.E.

In the melanocytic strain 1505b of Fig. 10 and melanocytic strain 10.3c of Fig. 11, hydrogen peroxide generation was assessed at 0, 30 and 45 minutes after UV irradiation. Addition of 1 nM CUV9900, NDP-MSH and MSH resulted in significant reduction of UV-induced hydrogen peroxide generation. This was found to be statistically significant from control as determined by ANOVA (p<0.05).

Significantly, at 45 minutes after irradiation treatment of the melanocytes by CUV9900 resulted in statistically significantly (ANOVA p<0.001) lower hydrogen peroxide generation in comparison with both NDP-MSH and MSH. However, at 30 minutes after irradiation in strain 1505b, MSH demonstrate less peroxide generation than NDP-MSH or CUV9900. This effect is most likely due to the lag-time observed in both melanocytes and keratinocytes required to generate hydrogen peroxide.

In conclusion, CUV9900 proved most effective at reducing the generation of hydrogen peroxide at 4.5 minutes in comparison to NDP-MSH and MSH. The efficacy of this potent MSH agonist to abrogate intracellular hydrogen peroxide is thought clinically significant when reviewing the mechanisms which lead to prolonged photodamage and mutagenicity of the melanocyte and keratinocytes.

### Example 6

### Measurement of UV-induced apoptosis by Annexin Va staining

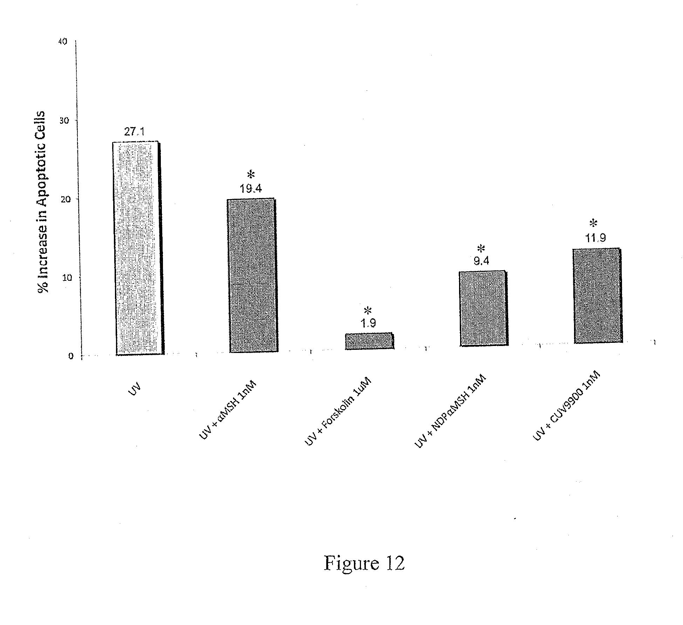

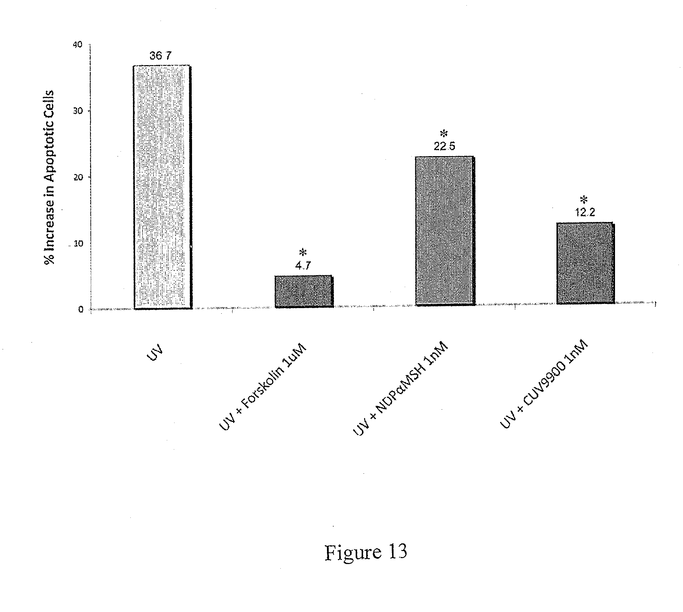

With reference to Fig. 12 and 13, melanocytes were maintained for the duration of the experiment in medium lacking bovine pituitary extract (BPE) to determine the survival effect of α-MSH or its analogs in the absence of the anti-apoptotic effect of TPA. Melanocytes were treated with 0, 1 nM α-MSH, 1 µM Forskolin, or 1 nM NDP-MSH or CUV9900 for 4 days prior to, and 24 h after exposure to a dose of 105 mJ/cm² UV, then stained for Annexin Va staining. The data are represented as percent increase above control, with triplicate dishes included in each group.

Comparison of the effect of CUV9900, NDP-MSH and MSH on the UV-induced apoptosis were made in the melanocyte strains 103c and melanocyte strain 1513c of Figs. 12 and 13, respectively. Both strains were treated with 1 nM CUV9900, NDP and MSH, respectively for 4 days prior and 1 day following irradiation. Floating as well as attached cells were harvested and stained for Annexin V, and analyzed by flow cytometry. In strain 103c, CUV9900 had an equal anti-apoptotic effect than NDP-MSH. This was found to be statistically significantly different from control as determined by ANOVA (p=<0.05).

### Example 7

### Ultrastructural visualization of DOPA-stained HM

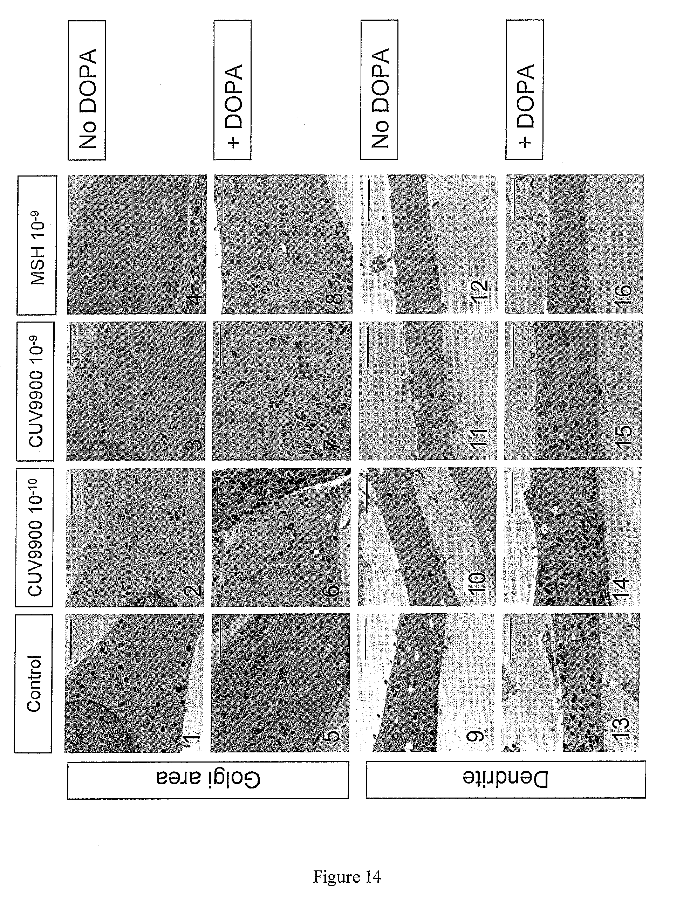

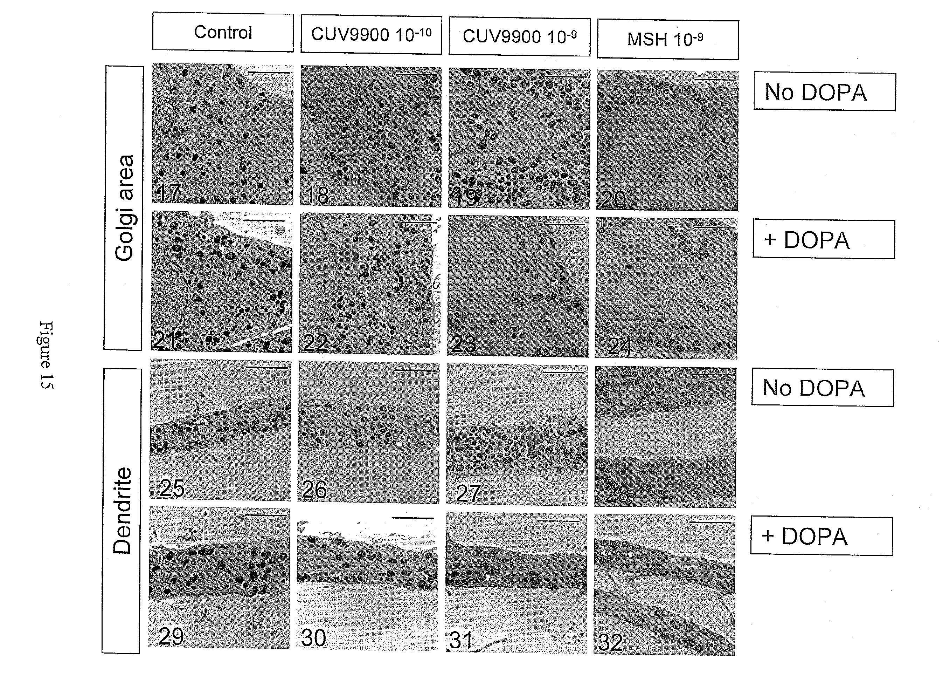

With reference to Figs. 14 and 15, two melanocytic strains, a lightly pigmented strain 1563c of Fig. 14 and strain 102b of Fig. 15 were plated in 4-well chamber slides and treated for four days with either 0.1 nM or 1 nM CUV9900 or 1 nM MSH for a total of 6 days. Cells were processed for electron microscopy in the presence or absence of histochemical reaction with DOPA. Microtome sections were analyzed in a JEOL JEM-1230 transmission electron microscope and photomicrographs (15,000 X) of the perinuclear area and dendrites were taken for each experimental group.

Treatment with either CUV9900, particularly at 1 nM, as well as with MSH resulted in increased mature (stage IV) melanosomes in the perinuclear area and more so in dendrites, and DOPA reactivity was increased in melanosomes (organelles), vesicles, endoplasmatic reticulum and trans golgi area.

In conclusion, the significant increase in melanosomes and melanin particles following the treatment with CUV9900 indicates the increase in activity of the pigment-producing organelle melanosome. Electronmicroscopically it is apparent that efficacy of cellular activity is achieved by minimal concentrations at 0.1 nM of CUV9900.

As noted, all the above experiments and examples were carried out using the Ac-Nle-Glu-His-D-Phe-Arg-Trp-NH₂ (SEQ ID NO: 1) variation of CUV9900 and whenever CUV9900 is used in the above examples said Ac-Nle-Glu-His-D-Phe-Arg-Trp-NH² is intended. Nevertheless, as also explained hereinabove the Ac-Nle-Gln-His-D-Phe-Arg-Trp-NH₂ (SEQ ID NO: 2) variation of CUV9900 would provide for substantially the same effects.

### Administration of CUV9900

CUV9900 of the invention can be administered to a subject using a variety of administration or delivery techniques known in the art. In the following, the description of the administration will refer to both variations of CUV9900, namely Ac-Nle-Glu-His-D-Phe-Arg-Trp-NH₂ (SEQ ID NO: 1) and Ac-Nle-Gln-His-D-Phe-Arg-Trp-NH₂ (SEQ ID NO: 2).

Advantageously CUV9900 may be administered in a sustained-release delivery system as described in International Patent Application No. PCT/AU2005/000181 (published as WO 2006/012667). The sustained release format can be an ocular insert, erodible microparticulates, swelling mucoadhesive particulates, pH sensitive microparticulates, nanoparticles/latex systems, ion-exchange resins and other polymeric gels and implants (Ocusert, Alza Corp., California; Joshi, A., S. Ping and K. J. Himmelstein, Patent Application WO 91/19481). These systems maintain prolonged drug contact with the absorptive surface preventing washout and nonproductive drug loss.

Advantageously, CUV9900 may be also administered topically using a transdermal delivery system as described in International Patent Application No. PCT/AU2005/001552 (published as WO 2006/037188). The topical transdermal delivery system includes (1) a peptide, (2) a solvent system in which the peptide is soluble, (3) optionally, a substance capable of in vivo stimulation of adenosine 3',5'-cyclic monophosphate (cAMP) or cyclic guanosine 3',5'-monophosphate (cGMP), and (4) optionally, a skin stabilizer.

In a preferred embodiment of the present invention, CUV9900 is administered to the subject using a sustained-release delivery system which enables CUV9900 to be maintained in the plasma of the subject for about 10 up to 14 days.

In a further preferred embodiment of the present invention, the amount of CUV9900 released into the plasma of the subject in the first 72 hours following administration is less than 60% of the total amount of CUV9900 administered to the subject.

It will be appreciated that the actual preferred amounts of CUV9900 in a specified case will vary according to the specific compounds being utilized, the particular compositions formulated, the mode of application, and the particular situs and subject being treated. Dosages for a given host can be determined using conventional considerations, e.g., by customary comparison of the differential activities of the subject compounds and of a known agent, e.g., by means of an appropriate conventional pharmacological protocol.

In certain embodiments of the present invention, the mode of administration will depend upon the subject to be treated and the peptide used. In various aspects, CUV9900 of the invention can be administered orally or parenterally. The term "oral" is used herein to encompass administration of the compounds via the digestive tract. The term "parenteral" is used herein to encompass any route of administration, other than oral administration, by which CUV9900 is introduced into the systemic circulation which include, but is not limited to, intravenous, intramuscular, subcutaneous, intraperitoneal, intradermal, ocular, inhalable, rectal, vaginal, transdermal, topical, buccal, sublingual, or mucosal administration. The term "mucosal" as used herein encompasses the administration of the compounds by methods that employ the mucosa (mucous membranes) of the human body such as, but not limited to, buccal, intranasal, gingival, vaginal, sublingual, pulmonary, or rectal tissue. The term "transdermal" as used herein encompasses the administration of the compounds that go into the skin or go through the skin using formulations such as, but not limited to, transdermal formulations, buccal patches, skin patches, or transdermal patches. The term "topical" as used herein encompasses administration by applying conventional topical preparations such as creams, gels, or solutions for localized percutaneous delivery and/or by solution for systemic and/or localized delivery to areas such as, but not limited to the eye, skin, rectum, and vagina.

In one aspect, delivery systems composed of devices or compositions containing CUV9900 can be manufactured that allow for the controlled-release, extended-release, modified-release, sustained-release, pulsatile-release, or programmed-release delivery of peptides in order to maintain concentration of the peptide in the plasma of the subject. Depending on the delivery system or composition of a formulation or route of administration chosen, drugs or active pharmaceutical ingredients can be delivered for hours, weeks, or months following a single administration. Drug-delivery devices include, but are not limited to pumps, needle-free injectors, metered-dose inhalers, and the like. Transdermal compositions with or without penetration enhancers include but are not limited to transdermal patches, microneedles, and transdermal formulations that achieve drug delivery using iontophoresis, sonophoresis, electroporation, thermoporation, perfusion, adsorption and absorption. Other delivery systems include, but are not limited to, biodegradable or non-biodegradable rods or other shaped implants, fibers, microparticles, microspheres, microcapsules, nanospheres, nanocapsules, porous silicon nanoparticles, in situ gelling formulations, in situ bolus forming compositions, quick dissolving tablets and the like, buccal patches, films, tablets, capsules, osmotic pressure driven formulations, liquid filled capsules, liposomes and other lipid based compositions and the like, pegylation and the like, hydrogel formulations, emulsions, microemulsions, and suspensions.

In one aspect, polymeric delivery systems can be microparticles including, but not limited to microspheres, microcapsules, nanospheres and nanoparticles comprising biodegradable polymeric excipients, non-biodegradable polymeric excipients, or mixtures of polymeric excipients thereof, or the polymeric delivery systems can be, but not limited to rods or other various shaped implants, wafers, fibers, films, in situ forming boluses and the like comprising biodegradable polymeric excipients, non-biodegradable polymeric excipients, or mixtures thereof. These systems can be made from a single polymeric excipient or a mixture or blend of two or more polymeric excipients.

A suitable polymeric excipient includes, but is not limited to, a poly(diene) such as poly(butadiene) and the like; a poly(alkene) such as polyethylene, polyethylene glycol (PEG), polypropylene, and the like; a poly(acrylic) such as poly(acrylic acid) and the like; a poly(methacrylic) such as poly(methyl methacrylate), a poly(hydroxyethyl methacrylate), and the like; a poly(vinyl ether); a poly(vinyl alcohol); a poly(vinyl ketone); a poly(vinyl halide) such as poly(vinyl chloride) and the like; a poly(vinyl nitrile), a poly(vinyl ester) such as poly(vinyl acetate) and the like; a poly(vinyl pyridine) such as poly(2-vinyl pyridine), poly(5-methyl-2-vinyl pyridine) and the like; a poly(styrene); a poly(carbonate); a poly(ester); a poly(orthoester) including a copolymer; a poly(esteramide); a poly(anhydride); a poly(urethane); a poly(amide); a cellulose ether such as methyl cellulose, hydroxyethyl cellulose, hydroxypropyl methyl cellulose, and the like; a cellulose ester such as cellulose acetate, cellulose acetate phthalate, cellulose acetate butyrate, and the like; a poly(saccharide), a protein, gelatin, starch, gum, a resin, and the like. These materials may be used alone, as physical mixtures (blends), or as co-polymers. Derivatives of any of the polymers listed above are also contemplated.

In one aspect, the polymeric excipient of the delivery system includes a biocompatible, non-biodegradable polymer such as, for example, a silicone, a polyacrylate; a polymer of ethylene-vinyl acetate; an acyl substituted cellulose acetate; a non-degradable polyurethane; a polystyrene; a polyvinyl chloride; a polyvinyl fluoride; a poly(vinyl imidazole); a chlorosulphonate polyolefin; a polyethylene oxide; or a blend or copolymer thereof.

In another aspect, the polymeric excipient includes a biocompatible, biodegradable polymer such as, for example, a poly(lactide); a poly(glycolide); a poly(lactide-co-glycolide); a poly(lactic acid); a poly(glycolic acid); a poly(lactic acid-co-glycolic acid); a poly(caprolactone); a poly(orthoester); a poly(phosphazene); a poly(hydroxybutyrate) or a copolymer containing a poly(hydroxybutyrate); a poly(lactide-co-caprolactone); a polycarbonate; a polyesteramide; a polyanhydride; a poly(dioxanone); a poly(alkylene alkylate); a copolymer of polyethylene glycol and a polyorthoester; a biodegradable polyurethane; a poly(amino acid); a polyetherester; a polyacetal; a polycyanoacrylate; a poly(oxyethylene)/poly(oxypropylene) copolymer, or a blend or copolymer thereof.

In one particularly advantageous aspect, the delivery system comprises an implant or rod, wherein the implant or rod comprises a biodegradable polymer, wherein CUV9900 is embedded within the implant or rod. In one particularly effective aspect, CUV9900 is encapsulated in an implant or rod composed of poly(lactide-co-glycolide), poly(lactide), poly(glycolide), or a mixture thereof. Lactide/glycolide polymers for drug-delivery formulations are typically made by melt polymerization through the ring opening of lactide and glycolide monomers. Some polymers are available with or without carboxylic acid end groups. When the end group of the poly(lactide-co-glycolide), poly(lactide), or poly(glycolide) is not a carboxylic acid, such as for example an ester, then the resultant polymer is referred to herein as blocked or capped. The unblocked polymer, conversely, has a terminal carboxylic group. In one aspect, linear lactide/glycolide polymers are used; however star polymers can be used as well. In certain aspects, high molecular weight polymers can be used for medical devices, for example, to meet strength requirements. In other aspects, low molecular weight polymers can be used for drug-delivery and vaccine delivery products where resorption time and not material strength is important. The lactide portion of the polymer has an asymmetric carbon. Commercially racemic DL-, L-, and D-polymers are available. The L-polymers are more crystalline and resorb slower than DL- polymers. In addition to copolymers comprising glycolide and DL-lactide or L-lactide, copolymers of L-lactide and DL-lactide are available. Additionally, homopolymers of lactide or glycolide are available.

In the case when the biodegradable polymer is poly(lactide-co-glycolide), poly(lactide), or poly(glycolide), the amount of lactide and glycolide in the polymer can vary. In one aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In one aspect, the biodegradable polymer can be poly(lactide), 85:15 5 poly(lactide-co-glycolide), 75:25 poly(lactide-co-glycolide), 65:35 poly(lactide-co-glycolide), or 50:50 poly(lactide-co-glycolide), where the ratios are mole ratios. The most preferred molar ratio is f 50:50 poly(lactide-co-glycolide).

In one aspect, when the biodegradable polymer is poly(lactide-co-glycolide), poly(lactide), or poly(glycolide), the polymer has an intrinsic viscosity of from 0.15 to 1.5 dL/g, 0.25 to 1.5 dL/g, 0.2.5 to 1.0 dL/g, 0.25 to 0.8 dL/g, 0.25 to 0.6 dL/g, or 0.25 to 0.4 dL/g as measured in chloroform at a concentration of 0.5 g/dL at 30 oC.

The amount of CUV9900 that is encapsulated or incorporated in the biodegradable polymer or in a liquid/gel-like substance will vary depending upon the selection of the biodegradable polymer, the encapsulation or incorporation technique, and the amount of CUV9900 to be delivered to the subject. In one aspect, the amount of CUV9900 encapsulated in the microcapsule, implant, or rod can be up to 50% by weight of the delivery system. In other aspects, the quantity and/or loading of CUV9900 encapsulated in the microcapsule, implant, rod or incorporated in the liquid/gel-like substance can be from 5 to 60, 10 to 50% (including 50%), 15 to 40%, or 15 to 30% by weight of the delivery system. The most preferred ratio of encapsulation or incorporation of CUV9900 is from 10% to about 50% (including 50%) in combination with a ratio of 50:50 poly(lactide-co-glycolide).

In another aspect, where CUV9900 is delivered by another delivery system such as a transdermal formulation, the amount of CUV9900 in the formulation can be from 0.001 to 10%, or 0.05 to 5% by weight of the formulation.

Other pharmaceutically-acceptable components can be encapsulated or incorporated in the delivery system in combination with CUV9900. For example, the pharmaceutically-acceptable component can include, but is not limited to, a fatty acid, a sugar, a salt, a water-soluble polymer such as polyethylene glycol (PEG), a protein, polysaccharide, or carboxmethyl cellulose, a surfactant, a plasticizer, a high- or low-molecular-weight porosigen such as polymer or a salt or sugar, or a hydrophobic low-molecular-weight compound such as cholesterol or a wax. In another aspect, the delivery system comprises an implant or rod, wherein CUV9900 is in the amount from 15% to 45% by weight of the implant or rod, wherein the rod or implant comprises poly(lactide) or poly(lactide-co-glycolide) such as, for example, 85:15 poly(lactide-co-glycolide).

Any of the delivery systems described herein can be administered using techniques known in the art. In one aspect, the delivery system can be administered subcutaneously to the subject. In this aspect, the duration of administration can vary depending upon the amount of CUV9900 that is encapsulated and the biodegradable polymer selected. In one aspect, the delivery system is administered subcutaneously to the subject and releases CUV9900 for a period of at least 2, 4, 6, 8, 10 or 12 days. In one aspect, the delivery system releases CUV9900 in the subject for up to three months. In various other aspects, the delivery system releases CUV9900 in the subject for 10 days, 15 days, 20 days, 25 days, or 30 days. In a particularly preferred aspect of the present invention, CUV9900 is administered at a constant level over a period of 10 up to 14 days.

In one aspect, any of CUV9900s can be combined with at least one pharmaceutically-acceptable carrier to produce a pharmaceutical composition. The pharmaceutical compositions can be prepared using techniques known in the art. In one aspect, the composition is prepared by admixing CUV9900 with a pharmaceutically-acceptable carrier. The term "admixing" is defined as mixing the two components together so that there is no chemical reaction or physical interaction. The term "admixing" also includes the chemical reaction or physical interaction between CUV9900 and the pharmaceutically-acceptable carrier.

Pharmaceutically-acceptable carriers are known to those skilled in the art. These most typically would be standard carriers for administration to humans, including solutions such as sterile water, saline, and buffered solutions at physiological pH.

Molecules intended for pharmaceutical delivery may be formulated in a pharmaceutical composition. Pharmaceutical compositions may include carriers, thickeners, diluents, buffers, preservatives, surface active agents and the like in addition to the molecule of choice. Pharmaceutical compositions may also include one or more active ingredients such as antimicrobial agents, anti-inflammatory agents, anesthetics, and the like.

Preparations for administration include sterile aqueous or non-aqueous solutions, suspensions, and emulsions. Examples of non-aqueous carriers include water, alcoholic/aqueous solutions, emulsions or suspensions, including saline and buffered media. Parenteral vehicles, if needed for collateral use of the disclosed compositions and methods, include sodium chloride solution, Ringer's dextrose, dextrose and sodium chloride, lactated Ringer's, or fixed oils. Intravenous vehicles, if needed for collateral use of the disclosed compositions and methods, include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, ointments, suppositories, sprays, liquids and powders. Conventional pharmaceutical carriers, aqueous, powder or oily bases, thickeners and the like may be necessary or desirable. CUV9900 can be admixed under sterile conditions with a physiologically acceptable carrier and any preservatives, buffers, propellants, or absorption enhancers as may be required or desired. Reference is made to documents cited herein, e.g., United States Patent No. 5,990,091,

WO 98/00166, and WO 99/60164, for the preparation of compositions for topical applications, e.g., viscous compositions that can be creams or ointments, as well as compositions for nasal and mucosal administration.

In the case when the composition is administered mucosally, ocularly, intranasally, or by inhalation, the formulation can be in the form of a drop, a spray, an aerosol, or a sustained release format. The spray and the aerosol can be achieved through use of the appropriate dispenser. The sustained release format can be an ocular insert, erodible microparticulates, swelling mucoadhesive particulates, pH sensitive microparticulates, nanoparticles/latex systems, ion-exchange resins and other polymeric gels and implants (Ocusert, Alza Corp. , California; Joshi, A., S. Ping and K. J. Himmelstein, Patent Application WO 91/19481). These systems maintain prolonged drug contact with the absorptive surface preventing washout and nonproductive drug loss.

## Claims

1. A compound having a structure selected from:
Ac-Nle-Glu-His-D-Phe-Arg-Trp-NH₂ (SEQ ID NO: 1); or
Ac-Nle-Gln-His-D-Phe-Arg-Trp-NH₂ (SEQ ID NO: 2)
or a pharmaceutically acceptable salt thereof.

2. The pharmaceutical composition comprising a compound of claim 1, or a pharmaceutically acceptable salt thereof, in combination with a pharmaceutically acceptable carrier.

3. The compound of claim 1 or a pharmaceutically acceptable salt thereof, or the composition of claim 2, for use in the repair of cellular DNA of dermal (skin) cells following UV irradiation in a mammal.

4. The compound of claim 1 or a pharmaceutically acceptable salt thereof, or the composition of claim 2, for use in the prevention of damage to cellular DNA of dermal (skin) cells following UV irradiation in a mammal.

5. The compound of claim 1 or a pharmaceutically acceptable salt thereof, or the composition of claim 2, for use in stimulating the synthesis of melanin in a mammal.

6. The compound of claim 1 or a pharmaceutically acceptable salt thereof, or the composition of claim 2, for use in the repair of cellular DNA of dermal (skin) cells.

7. The compound of claim 1 or a pharmaceutically acceptable salt thereof, or the composition of claim 2, for use in the prevention of damage to cellular DNA of dermal (skin) cells.

8. The compound of claim 1 or a pharmaceutically acceptable salt thereof, or the composition of claim 2, for use in treating cancer in a mammal.

9. The compound or a pharmaceutically acceptable salt thereof or the composition for use of claim 8 wherein the cancer is melanoma.

10. A rod, microcapsule, implant, gel or liquid formulation comprising:
a) the compound of claim 1; or
b) the composition of claim 2,
in combination with a biodegradable polymer, wherein the amount of the compound is from 5% to 60% by weight of the rod, microcapsule, implant, gel or liquid formulation.

11. The rod, microcapsule or implant of claim 10, wherein the amount of the compound is from 10% to 50% (including 50%) by weight of the rod, microcapsule, implant, gel or liquid formulation.

12. The rod, microcapsule, implant, gel or liquid formulation according to claims 10 or 11, wherein the polymer is poly(lactide-co-glycolide) having a mole ratio of lactide:glycolide of 50:50.

13. The compound or a pharmaceutically acceptable salt thereof or the composition for use according to claims 2 or 3 wherein the UV irradiation is UVA and UVB with a UVB:UVA ratio 75%:25%, the UV radiation being preferably one suitable for phototherapy in dermatology.

14. The compound or a pharmaceutically acceptable salt thereof or the composition for use according to claims 2 or 3 wherein the UV irradiation is one having a range and intensity suitable for phototherapy in dermatology.


SEQUENCE LISTING

<110>  Clinuvel Pharmaceuticals Limited

<120>  Hexapeptide with improved activity in the repair of cellular DNA
       of dermal cells

<130>  4559/PAF

<160>  4

<170>  PatentIn version 3.3

<210>  1
<211>  6
<212>  PRT
<213>  Artificial Sequence

<220>
<223>  CUV 9900 hexapeptide first variant


<220>
<221>  MOD_RES
<222>  (1)..(1)
<223>  Nle

<220>
<221>  MOD_RES
<222>  (1)..(1)
<223>  ACETYLATION

<220>
<221>  MISC_FEATURE
<222>  (4)..(4)
<223>  D-Phe

<220>
<221>  MOD_RES
<222>  (6)..(6)
<223>  AMIDATION

<400>  1

Xaa Glu His Phe Arg Trp
1               5


<210>  2
<211>  6
<212>  PRT
<213>  Artificial Sequence

<220>
<223>  CUV 9900 hexapeptide second variant


<220>
<221>  MOD_RES
<222>  (1)..(1)
<223>  Nle

<220>
<221>  MOD_RES
<222>  (1)..(1)
<223>  ACETYLATION

<220>
<221>  MISC_FEATURE

<222>  (4)..(4)
<223>  D-Phe

<220>
<221>  MOD_RES
<222>  (6)..(6)
<223>  AMIDATION

<400>  2

Xaa Gln His Phe Arg Trp
1               5


<210>  3
<211>  13
<212>  PRT
<213>  Artificial Sequence

<220>
<223>  alpha-MSH


<220>
<221>  MOD_RES
<222>  (1)..(1)
<223>  ACETYLATION

<220>
<221>  MOD_RES
<222>  (13)..(13)
<223>  AMIDATION

<400>  3

Ser Tyr Ser Met Glu His Phe Arg Trp Gly Lys Pro Val
1               5                   10


<210>  4
<211>  13
<212>  PRT
<213>  Artificial Sequence

<220>
<223>  NDP-MSH


<220>
<221>  MOD_RES
<222>  (1)..(1)
<223>  ACETYLATION

<220>
<221>  MOD_RES
<222>  (4)..(4)
<223>  Nle

<220>
<221>  MISC_FEATURE
<222>  (7)..(7)
<223>  D-Phe

<220>
<221>  MOD_RES
<222>  (13)..(13)
<223>  AMIDATION

<400>  4

Ser Tyr Ser Xaa Glu His Phe Arg Trp Gly Lys Pro Val
1               5                   10